## Europäisches Patentamt

## European Patent Office

## Office européen des brevets

(11) Veröffentlichungsnummer : **0 183 922**
**B1**

(12) **EUROPÄISCHE PATENTSCHRIFT**

(45) Veröffentlichungstag der Patentschrift :
24.01.90

(21) Anmeldenummer : 85110924.9

(22) Anmeldetag : 30.08.85

(51) Int. Cl.⁵ : **A 01 N 35/02**, A 01 N 27/00,
C 07 C 49/203 // (A01N35/02,
27:00)

(54) Verfahren und Mittel zur Bekämpfung des Rhombenspanners Boarmia rhomboidaria.

(30) Priorität : 05.09.84 DE 3432578

(43) Veröffentlichungstag der Anmeldung :
11.06.86 Patentblatt 86/24

(45) Bekanntmachung des Hinweises auf die Patenterteilung : 24.01.90 Patentblatt 90/04

(84) Benannte Vertragsstaaten :
AT CH DE FR IT LI

(56) Entgegenhaltungen :
Chemical Abstracts, Band 100, Nr. 25, 18.06.84; Seite 189, Ref.Nr. 204942b; Columbus, Ohio,US; J. W. WONG et al.:"Novel sex pheromone components from the fall cankerwrom moth, Alsophila pometaria." TETRAHEDRON LETTERS, Band 24, Nr. 49, Dez. 1983, Seiten 5505-5508; Pergamon Press Ltd., Oxford, GB - D. Becker et al.:"(3Z,6Z,9Z)-3,6,9-Nonadecatriene - A component of the sex pheromonal system of the giant looper, Boarmia (Ascotis) selenaria Schiffermüller (Lepidoptera: Geometridae)."
TETRAHEDRON LETTERS, Band 26, Nr. 4, Januar 85, Seiten 403-406, Pergamon Press Ltd., Oxford, GB - H.R. BUSER et al.:"(Z,Z)-6,9-Nonadecadien-3-one and (Z,Z,Z)-3,6,9-nonadecatriene: Identification and synthesis of sex pheromone components of Peribatodes rhomboidaria."
Chemical Abstracts, Band 102, Nr. 17, 29.04.1985, Seite 213, Ref.Nr. 144803m; Columbus, Ohio, US - G. SZOCS et al.: "A two-component sex attractant for males of the geometrid moth Alsophila quadripunctata."

(73) Patentinhaber : BASF Aktiengesellschaft
Carl-Bosch-Strasse 38
D-6700 Ludwigshafen (DE)

(72) Erfinder : Buser, Hans-Rudolf
Im Bungert 12
CH-8820 Waedenswil (CH)
Erfinder : Guerin, Patrick
Im Tobelacker 11
CH-8044 Gockhausen (CH)
Erfinder : Schmid, Augustin
Vuisse
1962 Pont-de-la-Morge (CH)
Erfinder : Francke, Wittko
Am Vorwerksbusch 3
D-2057 Reinbek (DE)
Erfinder : Arn, Heinrich
An der Halden
CH-8820 Waedenswil (CH)

**Beschreibung**

Der Rhombenspanner Boarmia rhomboidaria (= Peribatodes rhomboidaria Schiff. (gemmaria Brahm) = Boarmia gemmaria) aus der Familie der Spanner (Geometridae) ist in Europa, Rußland und Kleinasien verbreitet. Die polyphage Raupe tritt als Schädling an Obstbäumen und Reben auf.

Besonders in den letzten Jahren wird eine starke Zunahme der Fraßschäden an Rebknospen in verschiedenen Weinbauregionen beobachtet. (Englert, Deutsches Weinbaujahrbuch, 1979 ; Schmid et. al. : Revue suisse Vitic. Arboric. Hartic. 15, 1983). Der Rhombenspanner überwintert als Jungraupe im Boden, unter der Rinde des Stammes oder in Ritzen von Pfählen. Mit dem Ansteigen der Frühjahrstemperaturen erklettern die Raupen die Rebstöcke und fressen sich bereits beim Abheben der Knospenschuppen in die Knospen (Augen) hinein. Werden Teile der Knospe befressen, führen diese Schädigungen in Verlauf des weiteren Wachstums zu erheblichen Mißbildungen und zu Ertragsverlusten. Wird das Hauptauge abgefressen, kommt es zum Austreiben weniger fruchtbarer Beiaugen. Insbesondere bei Verzögerung des Rebenwachstums infolge kühler Witterung haben diese Knospenschädlinge schwere Ertragsausfälle verursacht.

Normalerweise treten zwei Faltergenerationen im Mai/Juni und im August/September auf. In den Tälern der Südalpen können sich von Ende Februar bis Mitte November unter günstigen klimatischen Bedingungen sogar drei Generationen entwickeln. (Forster-Wohlfahrt, Die Schmetterlinge Mitteleuropas, Band 5 (1981)).

Eine zuverlässige Methode zur Prognose des Auftretens dieses schädlichen Schmetterlings oder eine selektive Bekämpfungsmethode waren bisher nicht bekannt und damit eine gezielte Bekämpfung nicht möglich.

Die Erfindung betrifft ein Lockstoffpräparat zum selektiven Anlocken von männlichen Rhombenspannern.

Es ist an sich bekannt, daß bei Schmetterlingen von paarungsbereiten weiblichen Tieren Sexuallockstoffe (= Pheromone) erzeugt und in die Umgebung ausgeschieden werden ; männliche Schmetterlinge derselben Art können dann mit Hilfe dieses Riechstoffes die Weibchen auffinden.

Sog. Phermomonfallen, bestückt mit synthetischen Sexuallockstoffködern, werden in potentiellen Befallsgebieten ausgehängt. Der Fallenfang von männlichen Faltern erbringt den Nachweis zum Auftreten dieses Schädlings.

Außerdem lassen sich Hinweise zur Befallstärke und auf den richtigen Zeitpunkt der Bekämpfung ableiten.

Zur Bekämpfung dieser Schädlinge wurden bisher insektizide Wirkstoffe aus der Gruppe der Phosphorsäureester und Pyrethroide eingesetzt. Sämtliche derartige Produkte haben jedoch den Nachteil, daß sie keine selektive Wirkung zeigen und in vielen Fällen auch warmblütertoxisch sind.

Die bei der Anwendung der herkömmlichen chemischen Bekämpfungsmittel auftretenden Umweltschutzprobleme könnten bei der Bekämpfung von Schmetterlingen verringert oder behoben werden, wenn die Sexuallockstoffe auch zur Kontrolle eingesetzt werden.

Grundsätzlich gibt es drei verschiedene Möglichkeiten, Sexuallockstoffe im Pflanzenschutz anzuwenden :

durch Ausbringung von Lockstoffallen zum Nachweis eines Schädlings in einer bestimmten Region und zur Überwachung des Populationsverlaufes. Es handelt sich um ein wichtiges Hilfsmittel im integrierten Pflanzenschutz zur Bestimmung des günstigen Termines für eine Bekämpfung mit konventionellen Methoden (Monitortechnik)

durch Kombination eines Lockstoffes mit insektiziden Wirkstoffen. Es besteht die Möglichkeit, dem Köder/der Falle, Insektizide zuzusetzen oder aber nur in unmittelbarer Umgebung der Falle zu behandeln. Damit kann der größte Teil der aus weiter Entfernung angelockten männlichen Falterbevölkerung abgetötet werden (Abfangtechnik). Die Biotopbelastung ist auf ein vertretbares Maß reduziert

durch das Verfahren der Luftraumsättigung mit Sexuallockstoffen oder ähnlich wirkenden Substanzen. Die männlichen Schmetterlinge werden am Auffinden der Weibchen gestört und somit die Paarung der Tiere verhindert. In diesem Fall wird im gesamten Bereich der zu schützenden Pflanzenkultur eine größere Menge des Lockstoffes gleichmäßig im Luftraum verteilt, so daß die Männchen überall die Gegenwart des Duftstoffes empfinden können und ihr normales Orientierungsverhalten gestört ist.

Selbst bei dieser Verfahrensweise der Anwendung von Sexuallockstoffen werden nur verhältnismäßig kleine Mengen der Wirkstoffe, welche oft nur Bruchteile der üblichen Dosen der klassischen Insektizidwirkstoffe entsprechen, benötigt. (Birch (de.) : Pheromones North Holland Publ. Co. 1974).

Es handelt sich dabei um eine äußerst selektive, untoxische Bekämpfungsmethode unter größtmöglicher Schonung der Nicht-Zielorganismen, insbesondere der Nützlinge.

Es wurde nun gefunden, daß Zubereitungen, die (Z,Z)-6,9-Nonadecadien-3-on (II) ggf. in Kombination mit der Verbindung (Z,Z,Z)-3,6,9-Nonadecatrien (I) enthalten, einen wirksamen Lockstoff zur Anwendung der vorgenannten Methoden auf Boarmia rhomboidaria darstellen.

Gegenstand der Erfindung sind somit Mittel zur Anlockung oder zur Verwirrung der männlichen Tiere der Art Boarmia rhomboidaria, die gekennzeichnet sind durch einen Gehalt an Verbindung I oder Verbindung II oder vorzugsweise einem Gemisch der Verbindungen I und II in einem Mischungsverhältnis

1 : 100 bis 100 : 1, vorzugsweise 1 : 10 bis 10 : 1.

Die Verbindung I und ähnliche Verbindungen sind in anderen Schmetterlingen schon nachgewiesen.

(Z,Z,Z)-3,6,9-Nonadecatrien in Boarmia selenaria (Geometridae) (Becker et al. 1983 Tetrahedron Letters No. 24, 5505-5508) (Z,Z)-6,9-Nonadecadien in Bupalus piniarius (Geometridae) (Bestmann, Vostrowsky, 1983).

Die Verbindung II ist dagegen ein neuer, bisher unbekannter Stoff.

Auch die Mischung mit I, d. h. der Sexuallockstoff des Rhombenspanners, war bisher unbekannt (Klasen, Ridgway, Inscoe 1983, Chemical Attractions in Integrated Pest Management Programs ; Minks, 1984, Attractants and Pheromones in noxious insects).

Beide Komponenten sind EAD-aktiv und als Bestandteile des Pheromonbuketts beim Rhombenspanner anzusehen.

Die Herstellung von (Z,Z,Z)-3,6,9-Nonadecatrien (I) ist bekannt (D. Becker, et al, THL 1981, 5505).

(Z,Z)-6,9-Nonadecadien-3-on (II) kann wie folgt hergestellt werden (Schema 1).

Schema 1

Verbindung II

Nach Jain et al (J. org. chem. 48, 2266 (1983) wird aus 1-Bromnonan und 1-(2-Tetrahydropyranyloxy)-propin das 1-Bromo-3,6-hexadecadiin 1 hergestellt. Über eine Kollesynthese wird analog zu Mori's Carposina-Pheromon-Synthese Verbindung 1 in 1-Cyano-3,6-hexadecadiin 2 überführt. 3,6-Nonadecadiin-on-3 3 erhält man durch Grignard-Reaktion mit Ethylmagnesiumbromid, die nachfolgende Hydrierung mit Lindlar-Katalysator ergibt Komponente II.

(Siehe Schema 2 Seite 4 f.)

Schema 2

Verbindung II

Nach Schema 2 ergibt die Grignard-Kopplung von 1,4-Tetradecadiin (nach Bestmann et. al. THL 1983, 5505) mit 2-(2-Bromoethyl)-2-ethyl-1,3-dioxolan nach anschließendem Ansäuern 6,9-Nonadecadiin-3-on 3, das durch Chromatographie über eine $Al_2O_3$-Säule gereinigt und dann wie in Schema 1 in Komponente II überführt wird.

Zur anwendungsgerechten Formulierung des Wirkstoffs kommen sowohl flüssige wie auch feste Präparationen in Frage. Als Lösungsmittel kommen hochsiedende, aromatische, aliphatische oder cycloaliphatische Verbindungen in Betracht. Neben Kohlenwasserstoffen eignen sich Ester, Ether oder Ketone besonders gut. Typische Vertreter dieser Klassen sind z. B. : Xylol, Methylnaphthaline, Paraffinöle, Cyclohexanon, Ethylglykolacetat, Isophoron und Dibutylphthalat. Diese Lösungsmittel können allein oder in Mischungen mit anderen Komponenten Verwendung finden.

Weiterhin können Lösungen in pflanzlichen, tierischen oder synthetischen Ölen oder Fetten und anderen verdunstungshemmenden Lösungsmitteln mit niedrigem Dampfdruck (wie z. B. Dioctylphthalat) zum Zwecke der Wirkungsverlängerung hergestellt werden.

Des weiteren ist es möglich, den Wirkstoff in oder an natürliche oder synthetische feste Träger wie Gummi, Kork, Zellulose, Kunststoffe, gemahlene Kohle, Holzmehl, Silikate, Bimskies, gebrannten Ton oder ähnliche feste Trägerstoffe zu binden oder in speziellen Kapselformulierungen oder Kunststoffbehältern einzusetzen, um so eine gleichmäßige Abgabe an die Luft über längere Zeiträume hinweg zu erreichen. Außerdem kann der Wirkstoff aus geeigneten Behältern (Kapillaren oder anderen Gefäßen) durch enge Öffnungen zur Verdunstung gebracht werden, wodurch über längere Zeiträume hinweg besonders gleichmäßige Duftkonzentrationen erzielt werden, sowie aus mehrschichtigen Kunststoffplättchen, sogenannten Flakes.

Der Gehalt dieser Zubereitungen an Wirkstoff kann innerhalb weiter Grenzen schwanken. Generell kann das Verhältnis Wirkstoff : Zusatzstoff z. B. im Bereich von 10 : 1 bis 1 : $10^9$ liegen. In Kapselformulierungen oder anderen geeigneten Behältern kann z. B. der Wirkstoff in reiner, unverdünnter Form angewendet werden und sein Gewichtsanteil, bezogen auf die Gesamtformulierung, sehr hoch sein und bis zu 90 % betragen. Im allgemeinen genügen jedoch sehr geringe Wirkstoffkonzentrationen in den Zubereitungen, um die gewünschte Wirkung auf Boarmia rhomboidaria-Männchen auszuüben. Bevorzugt ist ein Mengenverhältnis Wirkstoff : Zusatzstoff von 1 : 10 bis 1 : $10^6$.

Die erfindungsgemäßen Mittel können in verschiedener Weise angewendet werden. z. B. kann man Fallen auslegen, die mit einer Formulierung desselben imprägniert sind. Hierdurch kann man die genaue Populationsdichte von Boarmia rhomboidaria bestimmen und, sobald diese eine kritische Grenze erreicht hat, eine Bekämpfung mit konventionellen Methoden durchführen.

Für diese Methode eignen sich am besten Formulierungen mit schwerflüchtigen Zusatzstoffen, die den Wirkstoff protrahiert abgeben, wie Gummi, Zellstoff, Wachse, Polymerisate oder verdunstungshemmende, schwerflüchtige Öle oder Paraffine, sowie Formulierungen in Kapseln oder anderen Behältern (Kapillaren), die den Lockstoff entweder durch ihre Wandung oder durch enge Öffnungen abgeben. Die Wirkstoffkonzentration liegt hier im allgemeinen im Bereich von 1 : 1 000 bis 1 : $10^6$.

Man kann ferner den Wirkstoff mit einem Kontakt- oder Fraßinsektizid kombinieren und auf diese weise die angelockten Männchen direkt vernichten. Die Wirkstoffkonzentrationen liegen hierbei in der gleichen Größenordnung wie oben.

Schließlich kann man den Wirkstoff auch in vergleichsweise hohen Konzentratrionen ausbringen, um die Männchen durch Desorientierung und Konfusion an der Paarung zu hindern. Hierfür werden im allgemeinen Formulierungen mit höheren Wirkstoffgehalten (10 : 1 bis 1 : 1 000) eingesetzt.

Die Wirksamkeit der erfindungsgemäßen Verbindung (II) wird durch folgende Screening-Ergebnisse belegt :

| Injizierter Gehalt an (Z,Z)-6,9-Nona-decadien-3-on | elektrisches Potential (mV) nach Reiz der Männchen-Antenne (EAD*) (Versuch Nr.) | | |
|---|---|---|---|
| | 1 | 2 | 3 |
| 70 µg | 2,95 | | |
| 7 µg | 1,95 | 4,9 | 4,35 |
| 700 pg | 0,95 | 4,15 | 3,25 |
| 70 pg | 0,35 | 2,75 | 1,95 |
| 7 pg | 0,08 | 1,0 | 0,9 |
| 700 fg | 0,02 | 0,3 | 0,3 |
| 70 fg | 0 | 0 | 0,1 |
| Blank | 0 | 0 | 0 |
| Weibchen-Extrakt (FE) aus 2 Tieren | 0,95 | 4,03 | 3,0 |

(Z,Z)-6,9-Nonadecadien-3-on ist in diesem Testsystem biologisch aktiver als (Z,Z,Z)-3,6,9-Nonadeca-trien.

*EAD (Elektroantennogramm-Test) siehe z. B. Arn et al, Z. Naturforsch. 30 c, 722 (1975); Hummel-Miller (ed.) Techniques in Pheromone Research.

## Patentansprüche

1. Mittel zur Bekämpfung des Rhombenspanners Boarmia rhomboidaria, enthaltend (Z,Z)-6,9-Nonadecadien-3-on (II) ggf. in Kombination mit der Verbindung (Z,Z,Z)-3,6,9-Nonadecatrien (I).

2. Mittel nach Anspruch 1, enthaltend (I) und (II) im Gewichtsverhältnis von 1 : 100 bis 100 : 1.

3. Mittel nach Anspruch 1, enthaltend (II) ggf. in Kombination mit (I) und übliche Zusatzstoffe und Hilfsmittel in üblicher Anordnung.

4. Verfahren zur Bekämpfung des Rhombenspanners, dadurch gekennzeichnet, daß man ein Mittel nach einem der Ansprüche 1 bis 3 in Verbindung mit einem Insektizid anwendet.

5. Verfahren zur Bekämpfung des Rhombenspanners, dadurch gekennzeichnet, daß man mit einem Mittel nach einem der Ansprüche 1 bis 3 das Auftreten des Rhombenspanners in an sich bekannter Weise feststellt und danach an sich bekannte Bekämpfungsmethoden anwendet.

6. Verfahren zur Beeinflussung der Vermehrungsrate des Rhombenspanners, dadurch gekennzeichnet, daß man ein Mittel nach einem der Ansprüche 1 bis 3 in einer solchen Menge anwendet, daß die männlichen Tiere der Art bei der Auffindung der weiblichen Tiere gestört werden.

7. (Z,Z)-6,9-Nonadecadien-3-on (II).

## Claims

1. An agent for controlling willow beauty (Boarmia rhomboidaria), containing (Z,Z)-6,9-nonadecadien-3-one (II) with or without (Z,Z,Z)-3,6,9-nonadecatriene (I).

2. An agent as claimed in claim 1, containing (I) and (II) in a weight ratio of from 1 : 100 to 100 : 1.

3. An agent as claimed in claim 1, containing (II) with or without (I) and customary additives and assistants in a customary arrangement.

4. A method for controlling willow beauty which comprises using an agent as claimed in any one of claims 1 to 3 in conjunction with an insecticide.

5. A method for controlling willow beauty, which comprises using an agent as claimed in any one of claims 1 to 3 for detecting the presence of willow beauty in a conventional manner and then employing a conventional control method.

6. A method for influencing the multiplication rate of willow beauty, which comprises applying an agent as claimed in any one of claims 1 to 3 in such an amount as to disrupt the search by the males of the species for females.

7. (Z,Z)-6,9-Nonadecadien-3-one (II).

## Revendications

1. Agent de lutte contre la phalène losangique Boarmia rhomboidaria, contenant du (Z,Z,Z)-3,6,9- nonadécatriène (I) et/ou de la (Z,Z)-6,9-nonadécadiène-3-one (II).

2. Agent selon la revendication 1, contenant (I) et (II) dans un rapport en poids de 1 : 100 à 100 : 1.

3. Agent selon la revendication 1, contenant (I) et/ou (II), ainsi que des additifs et adjuvants usuels dans la formulation habituelle.

4. Procédé de lutte contre la phalène losangique, caractérisé en ce qu'on applique un agent selon l'une quelconque des revendications 1 à 3 en combinaison avec un insecticide.

5. Procédé de lutte contre la phalène losangique, caractérisé en ce qu'on décèle de façon connue en soi, avec un agent selon l'une quelconque des revendications 1 à 3, la présence de la phalène losangique, puis on applique des méthodes de destruction connues en soi.

6. Procédé pour influer sur le taux de multiplication de la phalène losangique, caractérisé en ce qu'on applique un agent selon l'une quelconque des revendications 1 à 3 dans une quantité telle que les animaux mâles de l'espèce soient perturbés lorsqu'ils trouvent les animaux femelles.

7. (Z,Z)-6,9-Nonadécadiène-3-one (II).